# EUROPEAN PATENT APPLICATION

(11) **EP 1 656 893 A2**
(43) Date of publication of application: **17.05.2006**
(21) Application number: 05253156.3
(22) Date of filing: 23.05.2005
(51) Int. Cl.: A61B 17/28, A61B 17/32

(54) **Combined grip-cut tool**

(30) Priority: 24.05.2004 GB 0411520
(71) Applicant: Khan-Sullman, Russell, London E6 3BA (GB)
(72) Inventor: Khan-Sullman, Russell, London E6 3BA (GB)

(57) **Abstract**

A Grip-Cut Tool comprises two shear bodies co-acting via a means of motion (e.g. a pivot hinge), wherein each shear body incorporates a cutting edge blade, a means of handling and an integral gripping/holding component, the two shear-bodies being arranged such that the active gripping surfaces of the first body are complementary to and fit against the active gripping surfaces of the second body when the tool is in the closed position, the Gripcut tool being characterised in that at least part of the gripping surface/component of the first shear body intersects with and exists in the same plane containing at least part of the cutting edge of the first shear body (i.e. all or part of the cutting edge of the first shear body borders/acts as a boundary to part of the gripping component of the first shear body), and further characterised in that no part of the cutting edge of the second shear body intersects with, or acts as a boundary to the gripping component of the second shear body.

## Description

### BACKGROUND

It is desirable sometimes when cutting something to bold it so that it does not fall on the floor, but with one hand actioning the cutting, and the other gripping part of the object to be cut, the remaining cut part cannot be held.

An example of this is where a Dental technician is cutting a length of orthodontic wire. One part usually drops, as it is not always easy to grip both part with the same hand.

Another, preferred, example of a situation in which it would be desirable to be able to grip the object being cut is in the use of suture scissors. Currently, sutures are removed by first cutting the suture with one instrument (scissors), and then removing the cut suture via a second instrument (i.e. a pair of tweezers) Suture removal therefore requires two actions, using two different instruments.

Preferable embodiment of this device involve the provision of a Gripcut tool that both cuts and grips one of the two cut ends of the suture at the same time, allowing suture removal in a single action, single-handedly and with a single Instrument, as the Gripcut tool gripping one cut end of the suture is removed from the field of operation, thereby saving clinical time and effort, and also the expense of having to use, and sterilise, two separate tools.

There is therefore the need for a tool that grips and cuts an object in a single action.

### DESCRIPTION AND TECHNICAL INFORMATION

A grip-cut tool having two mutually co-acting component shear bodies (wherein each shear body incorporates a cutting edge blade and a gripping surface on a jaw/shank, and a lever arm with a loop handle, or other means of handling), pivotally connected together by a means of motion (e.g. a hinge), wherein the plane containing the longitudinal axis of the upper border (bearing and containing then cutting edge) of the jaw/shank of the first shear body traverses/intersects the gripping surface of the first shear body, and where the plane containing the longitudinal axis of the upper border (bearing and containing the cutting edge) of the jaw/shank of the second shear body does not traverse or intersect with the gripping surface of the second shear body but instead extends at an angle to the plane of the shear body, such that when the device is in the closed position, the gripping surfaces of the co-acting shear bodies fit together, and are complementary to one another, facilitating gripping and cutting when the tool is moved from the open position to the closed position.

The device is designed such that the two co-acting gripping surfaces face towards one another.

The form of the device is such that the active gripping surface of the first shear body fits against and is complementary to the active gripping surface of the second opposing, co-acting shear body (the two active gripping surfaces coming into contact when the tool is in the closed position),

In one preferable form, the gripping surfaces of the gripping members exist on curving or rounded planes, so that the gripping surface on one gripping member is convex, and fits into and against the concavity bearing the second gripping surface located on the second gripping member. The gripping surfaces may also be flat, rectilinear, non-rectilinear or rounded, or a combination of these types.

The preferable form of this invention would involve part of the length of one shear body acting as (at least in part) the gripping member, such that the gripping surface on that shear body exists along the upper, cutting edge border of the shear body, and wherein the boundary of that gripping surface of that shear body comprises part or all of the following: - the cutting/blade edge blade side of the upper border of the shear body, the non-cutting edge blade side of the upper border of the shear body (or an extension there from), and having a tip-end and a rear-end

Therefore, the depth between the non-cutting edge side and the cutting edge side of the upper border of the jaw of one of the shear bodies may contain all or part of the gripping surface of that shear body; whilst the co-acting, second shear body bears a gripping member that extends at an angle from the longitudinal plane of the second shear body, designed to fit into and against the gripping surface located, at least in part, in the upper border of the first shear body.

In this, then, the longitudinal axis of one shear body extends through the gripping surface of its associated gripping part, whilst the longitudinal axis of the second shear body does not extend through the gripping surface of its associated gripping member.

Further, the level of the first shear body's gripping surface intersects with, at least in part, the level of the cutting edge in the first shear body, whilst the plane and level of the second shear body's gripping surface is separate and different from the level of the cutting edge of the second shear body. In the first shear body the gripping surface is located along part of the upper border of the shear body, whilst in the second shear body the gripping surface is located at some point or level below the upper (cutting edge) border and above the lower (non-cutting edge) border.

Thus, where one shear body contains a notch or other feature bearing a gripping surface, the other, opposing shear body carries a gripping member complementary to and designed to fit into and against that notch when the tool is closed.

Another preferred example of the device could use a triangular shaped tooth that fits into a V-shaped notch or recess. Other shapes or styles of gripping 'tooth' on one gripping member can be designed to fit into complementary shaped recesses in the second gripping member.

Further embodiments may provide more than one gripping surfaces on each gripping member wherein each gripping surface may have parts that exist in different planes, and at an angle to one another. In all cases, the gripping surfaces fit snugly against one another when the device is in the closed position.

Such an arrangement allows the simultaneous or almost simultaneous cutting and gripping of a material at one time. Both parts of the cut object may be gripped if gripping surfaces are provided on both planar sides of the tool, i.e. mounting gripping members on both sides of the longitudinal plane containing the plane of the shear bodies) device and on either side of the blade edge axial length. In this arrangement when the tool is in the closed position, it grips the material that has been cut on both sides of the cut.

The blade edges are arranged to operate against each other to action a cutting action, as exemplified in the cutting action performed by a pair of scissors as the lever arms are brought to a closed position.

The pair of lever arms may be rotatably joined via a pivot to allow the shear bodies to move from an open position to a closed position via an arcuate path. The two gripping members may be also being incorporated into a resilient, tweezers-like tool.

Such a tool may also find further use in the food world, as the actions of knife and fork are combined into one.

This arrangement allows the simultaneous cutting and gripping of an object in a single closing action with a single tool, single-handedly.

The blades may pivot about a common pivot point.

A pair of co-acting gripping jaws mounted to one side of and not in the same plane as the plane containing the longitudinal axis as the blade edges (but where the axis of the length of the gripping members may be parallel to the axis of the length of the blade edges), each gripping jaw being mounted on all or part of the shear body such that the gripper components/jaws and the cutting edge blades are brought together when the shear bodies are moved from the open position to the closed position.

### Brief description of the drawing

By way of example one embodiment of the invention will now be described with reference to the accompanying drawings in which:
Figure 1 is a perspective view of a Gripcut Tool when in a scissor-like arrangement according to a first embodiment of the invention,
Figure 2 shows at (A) a perspective view of the second embodiment of the invention, and at (B) the embodiment in an open position,
Figure 3 shows at (A) another embodiment (in this case the 'Twissors' embodiment that can be provided for use in the clinical or artistic field) of the invention, and at (B) the embodiment when in the open position,
Figure 4 shows at (A), (B) and (C) views of the embodiment shown in Figure 3(A), in the closed position, in the three mutually orthogonal directions marked respectively X, Y ad Z of Fig. 3(A),
Figure 5 shows at (A) yet another embodiment of the invention in the closed position, and at (B) in the open position.
Figure 6 shows a further embodiment at (A) when closed and at (B) when open.

### Detailed Description of example of the Invention

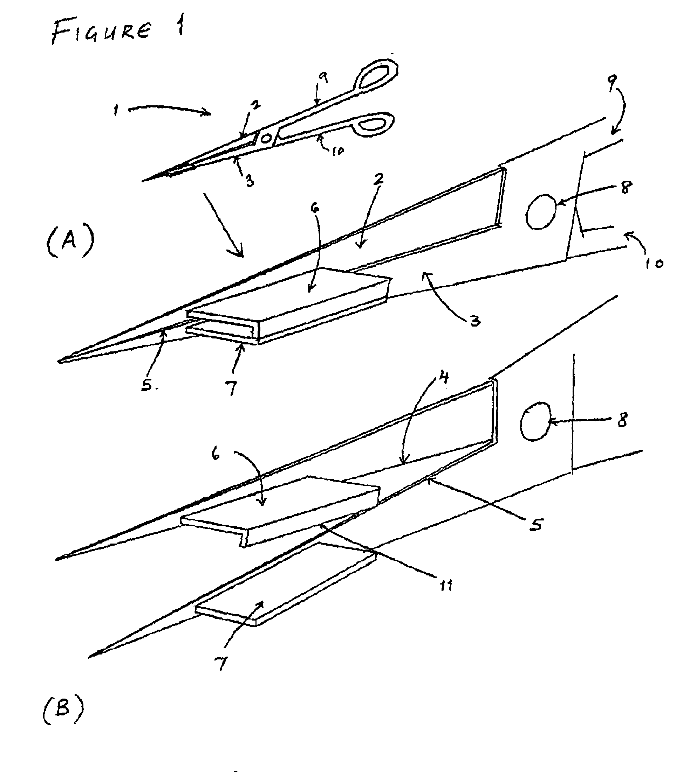

The Grip-cut tool 1 of Figure 1(A) has component bodies 2 and 3 with cutting edge blades 4 and 5 respectively, with body 2 mounting gripping member 6 and body 3 mounting gripping member 7. The whole pivots via hinge 8, the action of opening and closing facilitated by the movement of lever arms (loop handles) 9 and 10. Contact of element 11 of member 6 against member 7 actions the gripping when the tool is placed in the closed position. This closing action also causes the blades 4 and 5 to co-act in a cutting motion that will cause any material placed between the blades 4 and 5 to be cut.

Considering body 2 to be exemplary, it can be seen that the longitudinal axis of member 6 and the plane of 11 are not the same as plane and axis of the body 2.

Opening and closure of handles 9 and 10 causes an arcuate movement of bodies 2 and 3 respectively, with resultant opening and closure of 2 and 3 and also 6 and 7.

Figure 1(B) shows the tool in the open position, whereas 1(A) showed the tool in the closed position.

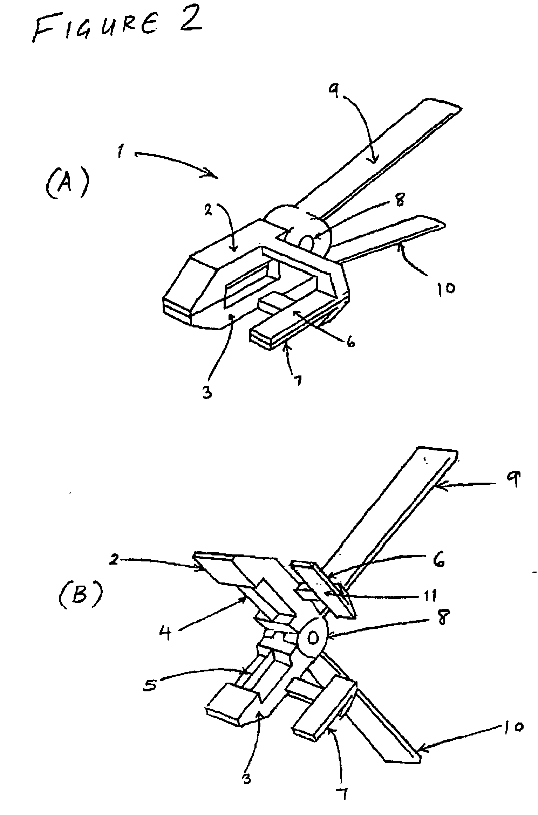

Figure 2 shows a second embodiment of the invention, with it closed in Fig 2(A) and open at Fig 2(B), of the type that can be used to cut flex, steel wire, etc. like parts of the embodiment of Fig 2(A) have like reference numerals to the embodiment shown in Figure 1. This tool also opens and closes via a pivoting hinge.

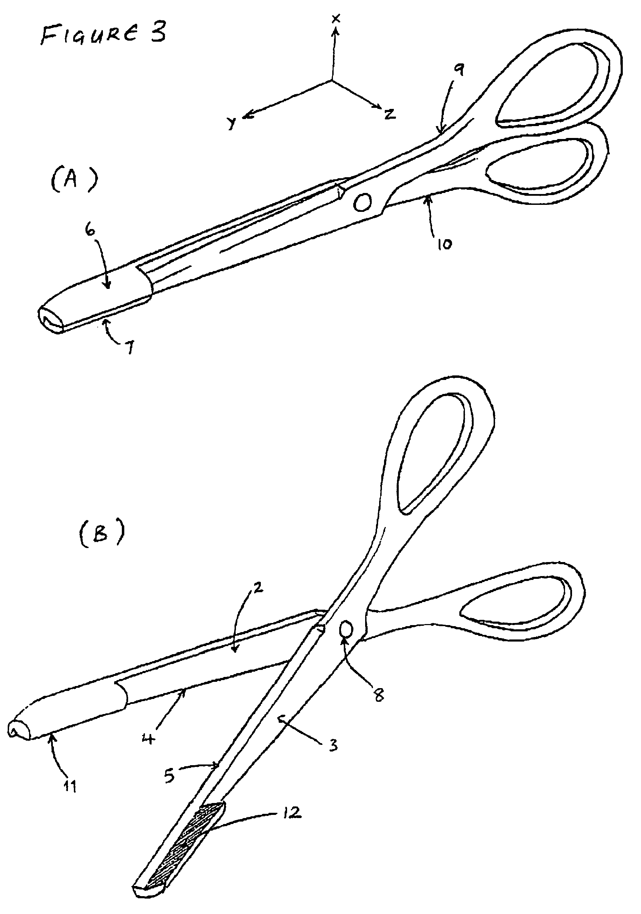

Figure 3 shows a preferred embodiment of the invention for suture removal. 3(A) shows the tool in the closed position, and at (B) in the open position, with the serrated area 12 being the grip surface against which element 11 comes into contact when the tool is in the closed position, allowing the object being cut to be gripped firmly during and after cutting.

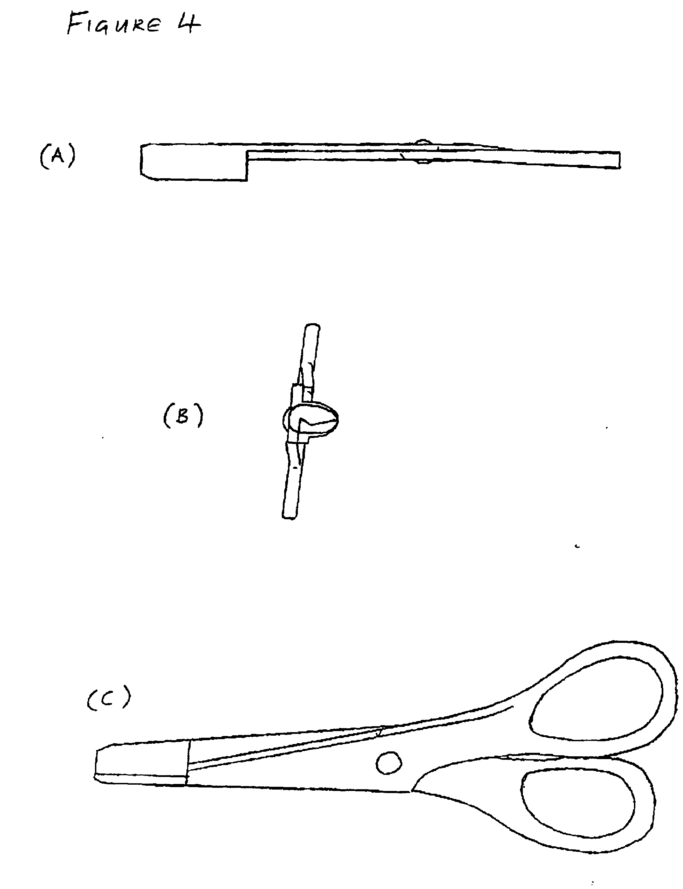

Figure 4 shows at (A), (B) and (C) views of the embodiment shown in Figure 3(A) from each of the three mutually orthogonal directions X, Y and Z that are marked in Fig. 3(A).

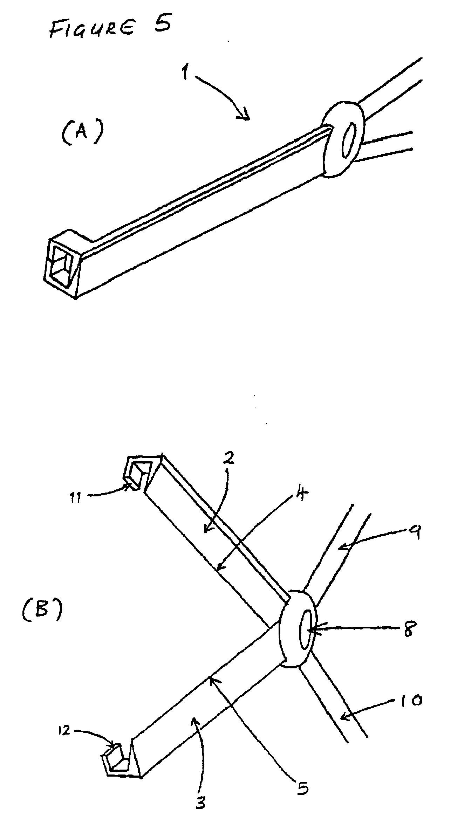

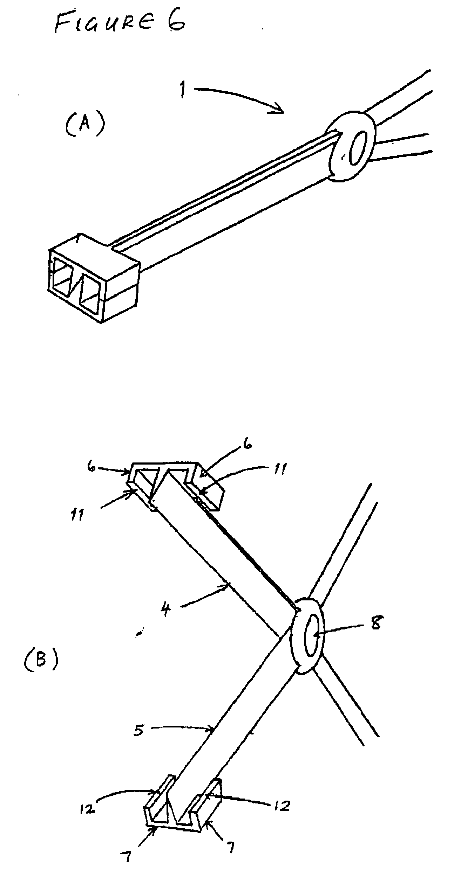

Figure 5 shows a stylised embodiment of the invention in the closed position at (A) and in the open position at (B).

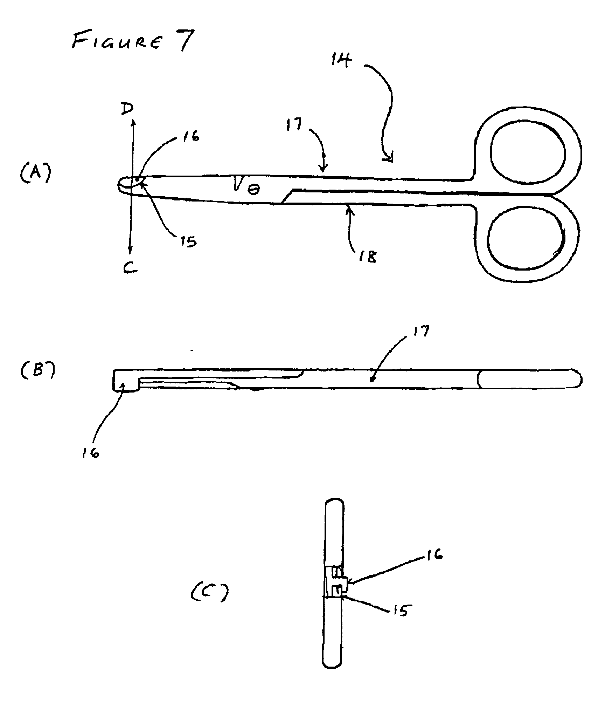

Figure 6 shows yet another stylised embodiment of the invention wherein there are provided two pairs of gripping members 6 and 7 on either planar side of the invention, so that gripping surfaces 11 and 12 exist on both sides of the longitudinal axis of the cutting blades 4 and 5, allowing both cut ends of the object being cut to be gripped.

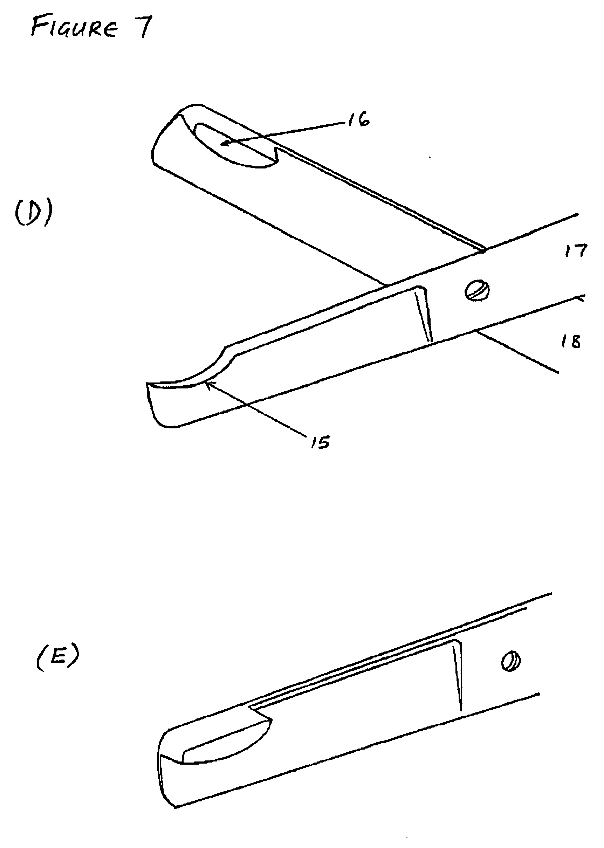

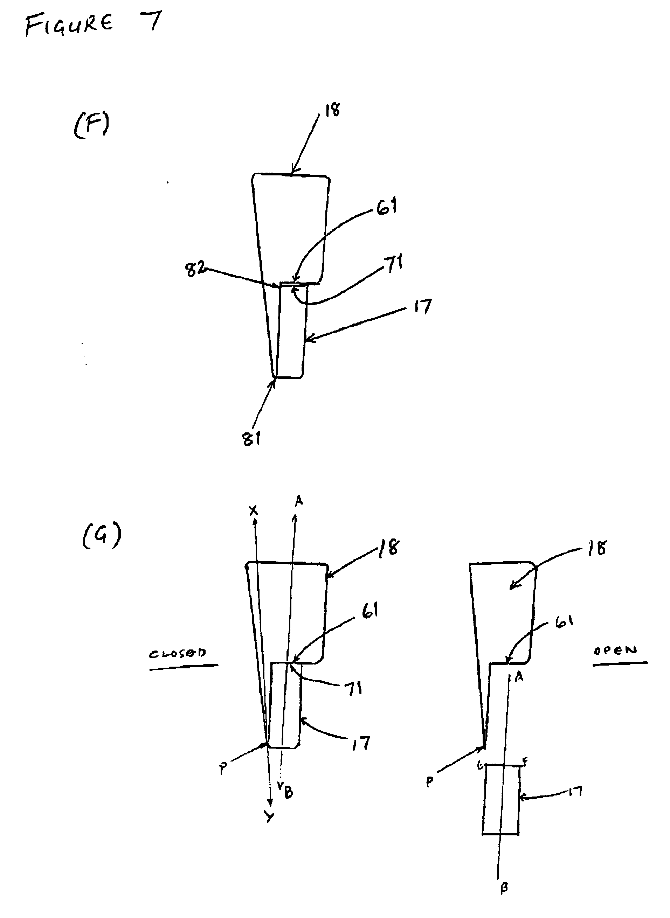

Figure 7 shows a plan view of yet another, preferred, embodiment of the invention (wit 7A, 7B and 7C showing views of this embodiment from one side, from above, and from the front respectively) when incorporated into a notched style of suture scissors 14. In this embodiment the concave notch 15 borne on the upper, cutting-edge, border of one shear body 17 is modified to bear a gripping surface (such that the gripping surface existing in a concave plane), with a complementary convex gripping element 16 (with a gripping surface arranged on a curved and convex plane) incorporated into the other, opposing, co-acting, shear body 18.
Figure 7 (D) and 7(E) show perspective views of the embodiment of the invention shown in plan views of Figure 7(A), (B) and (C), when in the open and closed position respectively.
Figure 7(F) shows a cross-section view of the embodiment shown in Figure 7(A) when viewed at a sliced section CD. It can be seen that gripping surface 61 of shear body 18 meets with gripping surface 71 of shear body 17 when the device is closed as shown. The cutting edge located at the upper border of 17 and 18 are numbered 82 and 81 respectively.
Figure 7(G) shows that the plane AB containing the longitudinal axis of the upper border EF of shear body 17 intersects with the gripping surface 71 of body 17, whilst the plane XY containing the plane of the upper border P of the shear body 18 is separate and different and does not intersect with the gripping surface 61 of 18.

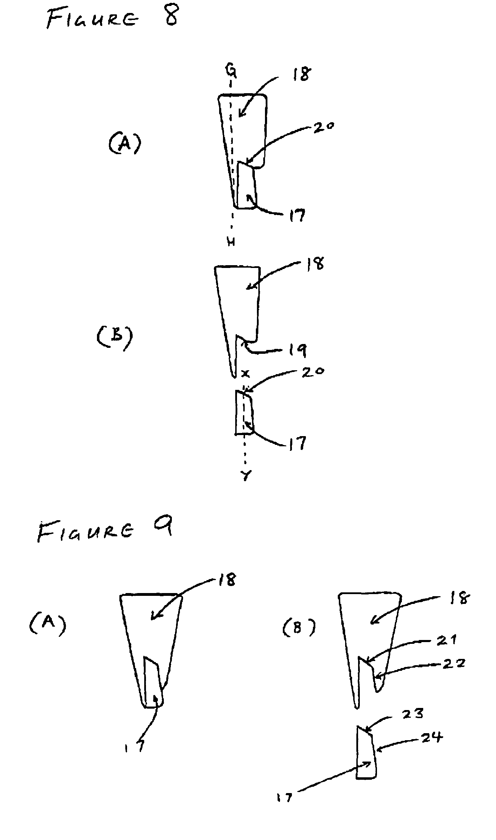

Figure 8(A) and 8(B) show a cross-section view of the shear bodies 17 and 18 when viewed in cross-section at plane DC in Figure 7(A), when closed and when open respectively, with gripping surface 19 located on shear body 18 and gripping surface 20 located on shear body 17 show a perspective view of the embodiment shown in Figure 7 when in the open and closed positions respectively. The gripping surface 20 exists on the upper cutting-edge border of shear body 17, and is intersected by the plane XY of the longitudinal axis, whereas the gripping surface 20 of shear body 18 does not intersect with the plane GH containing the longitudinal axis of the shear body.

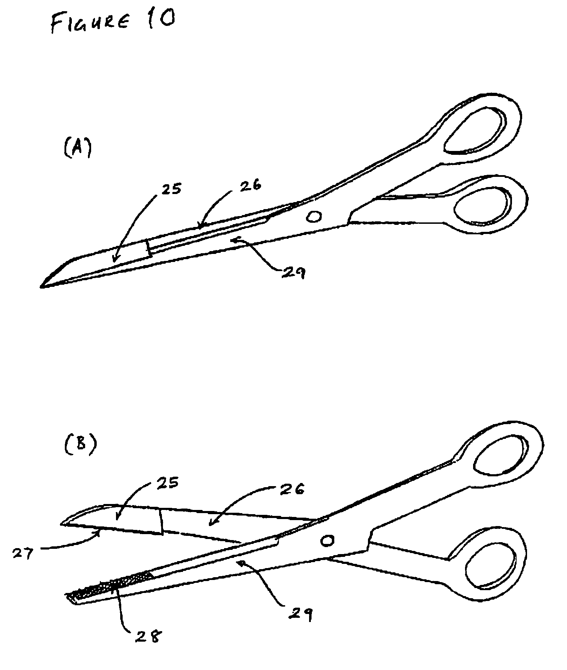

Figure 9 shows a further cross-sectional embodiment of the invention wherein the gripping surfaces extend over two or more surface, with shear body 18 bearing gripping surfaces 21 and 22, whilst shear body 17 bears gripping surfaces 23 and 24.

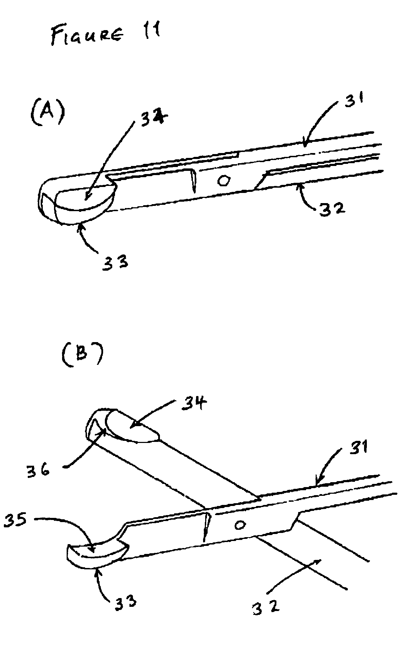

Figure 10(A) and 10(B) shows another scissors-like embodiment of the invention when closed and open respectively, wherein the gripping member 25 located on shear body 26 and incorporating gripping surface 27 grips against the gripping surface 28 located on the upper border of the shank of the shear body 29.

A further embodiment of this invention when part of a notched suture scissors manifestation requires only one gripping member 16, on the non-notched shear body 18, as the upper border of the notch 15 on the notch-bearing shear body 17, acts as the opposing gripping element, as shown in Figure 7.

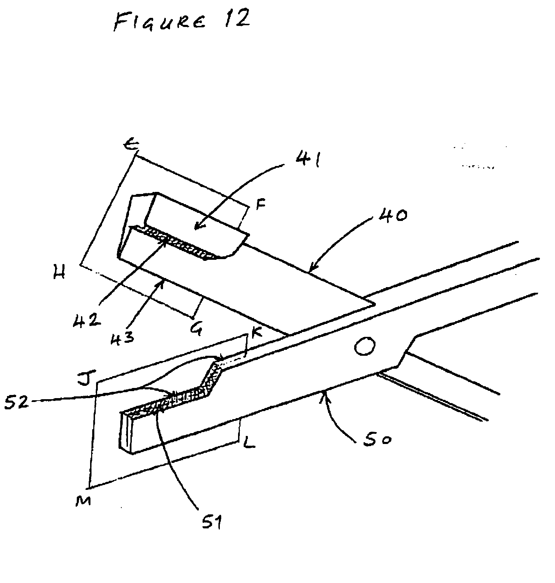

Figure 11 shows at 11(A) and 11(B) another embodiment of a scissors-like embodiment of the device when close and open respectively, wherein the gripping members 33 and 34 extend at an angle away from the shear bodies 31 and 32 respectively, and each bearing gripping surface 35 and 36 respectively.
Figure 12 shows a stylised version of the device to illustrate the important features being claimed for, wherein: -

Shear body 40 bears gripping member 41 with gripping surface 42 and cutting-edge 43, wherein the plane of the cutting edge blade EFGH does not intersect with the gripping surface 42;
And where shear body 50 bears a gripping surface 51 located at the upper (cutting-edge) border 52, with the plane of the cutting edge blade JKLM intersecting with and involving the gripping surface 51.

Similarly, when the unmodified notch is being used as one of the gripping elements, the gripping element located on the non-notched shear body has a gripping surface that grips against the upper border of the notch and the non-adjacent blade side of the shear body. i.e. the gripping surfaces on the notched shear body are the surfaces of the upper border of the notch and part of the side of the shear body that does not touch with or contact with the opposing co-acting shear body (the side of the shear body that is not adjacent to the other co-acting shear body). In this way both shear bodies have gripping elements in which the gripping surfaces of each exist in two or more planes. This is shown in Figure 10.

Other methods of gripping elements may incorporate gripping members with rounded or domed gripping surfaces that fit into a complementary dish-like hollow, a V-shaped notch bearing gripping surfaces designed to accept and be complementary to the gripping surfaces on a triangular tooth that intimately engages with the V-shaped notch, other types and styles of toothed elements complementary to and designed to fit into complementary recesses, or other types of gripping, co-acting, member.

A plurality of co-acting blades and gripping member arrangements may be provided on a single device.

From the foregoing it will be appreciated that embodiments of the invention may incorporate one or more of the following features:
1. A Grip-cut tool comprising a pair of jaws (each carrying a cutting edge blade), each jaw being rigidly attached to a lever arm or other means of handling, with a gripping member being integrally and inflexibly incorporated and mounted along part or all of the length of each of the cutting edge blade jaws, adjacent to the cutting edge blade on the jaw contained on the component to which they are mounted, with the blades being co-acting against one another and the gripping members also being co-acting against one another, the gripping surfaces on the gripping members being designed to be complementary to one another and to fit against one another (the gripping surfaces being preferably arranged on curved and/or flat planes), the opening and closure of the handles actioning the opening and closure of the tool;
2. The plane of the gripping surface of one shear body is intersected by the longitudinal axis of the shear body to which it is connected, and the plane of the gripping surface of the second co-acting shear body is not intersected by the longitudinal axis of the second shear body.
3. The gripping surfaces of each co-acting gripping member can exist in more than one plane, each plane at angles to one another,
4. The relationship between the gripping member and the cutting blade on each shear body is fixed,
5. The gripping members may be mounted along part or all of the shanks of the device;
6. The gripping surfaces of each co-acting gripping member can exist in curved, rounded and/or non-rectilinear planes fitting against each other and complementary to one another to facilitate gripping.
7. The blades, gripping members and means of handling not being in .line and not sharing the same longitudinal plane or the same longitudinal axis;
8. The lever arms may be rectilinear or non-rectilinear;
9. More than one means of motion may be incorporated;
10. The tool may be provided with a protective cover or sheath;
11. The gripping members exist in an arrangement such that when the tool is moved from the open position to the closed position the active gripping surfaces of the gripping members come into contact with one another to allow gripping.
12. The gripping surfaces being integrally and rigidly incorporated into their respective shear bodies;
13. The device may be provided with a gripping member provided on only one of the two shear bodies, intended to engage with part of the existing or current structure of the second shear body, wherein that part of the second shear body acts as a gripping surface complementary to the gripping member of the first shear body;
14. There is no flexibility or movement between the gripping surface and the cutting edge on each shear body during function or use;
15. The gripping surface adjacent parts of the gripping members being vertically orientated in relation to the plane of the shear-bodies;
16. The gripping surfaces on one gripping member being essentially similar but opposite to the gripping surfaces on the second gripping member to allow a snug and close fit of one against the other;
17. The gripping members may be incorporated into a resident tweezers-like arrangement;
18. The gripping surfaces of the gripping members come into contact with one another when the device is in the closed position:
19. A means of locking the device in the closed position can be provided,
20. The gripping surfaces of the gripping members come into close proximity with one another when the device is in the closed position,
21. Part or all of the tool may have a longitudinal axis that is the same as, similar to, or generally parallel to the pivot axis;
22. The handles may be located on the same side as the blade-gripping member combination, or may exist on the opposite side to the blade-grips combination;
23. The handles may be joined directly to the jaws;
24. The jaws (i.e. the gripping members-cutting blades combination) may overlie one another, or alternatively, be side by side;
25. The gripping surface component of the gripping members complementary to one another may be shaped or patterned to optimise and facilitate satisfactory gripping;
26. The device may be provided with only one specialised gripping member on the first shear body, the action of the other missing member to be facilitated by another structure, previously-existing feature or other structural/style modification on the other, second, opposing shear body. e.g. a single gripping member on one shear body designed to fit against the previously-existing notch on the opposing shear body in a pair of notched suture scissors;
27. The device may be provided in an embodiment in which there exist a plurality of co-acting blades and integral gripping member arrangements incorporated into a single device to allow simultaneous cutting and gripping at one time along an object to be cut

Other modifications and embodiments of the invention which will be readily apparent to those skilled in this art, are to be deemed to be within the scope and ambit of the invention, and the particular embodiments hereinbefore described may be varied in construction and detail, e.g. interchanging (where appropriate or desired) different features of each, without departing from the scope of the patent monopoly hereby sought.

## Claims

1. A Grip-cut tool having two component shear bodies connected together via a means of motion (e.g. a pivot hinge), each shear body comprising a cutting edge blade, a jaw/shank and a lever arm with loop handles (or other means of handling), wherein a gripping/holding member is integrally, inflexibly and rigidly incorporated into each component shear body's jaw/shank and arranged so that the active clamping parts or gripping surfaces of the two opposing co-acting gripping members face each other and fit against one another when the tool is closed, and where the active gripping surface of a gripping member on one component shear body is complementary to the active gripping surface of the gripping/holding member mounted on the opposing, co-acting component shear body, and **characterised in that** the gripping surface of one shear body is intersected by the plane containing the longitudinal axis of the upper (cutting edge) border of that shear body, whilst the gripping surface of the second (opposing and co-acting) shear body is not bisected or intersected by the plane of the longitudinal axis of the upper (cutting edge) border of the second shear body; and where the gripping surface of one shear body is located (at least in part) at the same or a similar level to the upper cutting edge border of that shear body, whilst the gripping surface of the second shear body extends at an angle from the plane of the jaw of the second shear body at a point between the upper cutting edge border and the lower non-cutting edge border;

2. As claimed in claim 1 wherein the gripping members exist on one or both planar side(s) of the device or of the cutting blades (thereby allowing gripping to occur on one or both sides), such that the longitudinal plane and longitudinal axis of the gripping members is different to the plane and axis of the longitudinal length of the cutting blades, and the blade and gripping member are not arranged in line one behind the other;

3. As claimed in claim 1 and 2 wherein the planes of the gripping surfaces of the gripping members may be flat, curved in one or more planes, arcuate, angled, non-rectilinear, rounded, some other shape, or a combination of these, and complementary to one another,

4. As claimed in any preceding claim wherein one of the two shear bodies is notched or otherwise shaped to accept the gripping member of the opposing shear body when the tool is in the closed position;

5. As claimed in any preceding claim wherein the gripping member of one shear body is located between the upper (cutting-edge) border and the lower (non-cutting edge) border of that shear body, so as to allow contact of its gripping surface with that of the opposing shear body when the tool is closed;

6. As claimed in any preceding claim wherein the gripping surfaces of one or both of the shear bodies extend at an angle to the plane of the longitudinal axis of the shear body(s), said angle being less than, greater than, or the same as, 90 degrees, or a combination of these;

7. As claimed in any preceding claim wherein the blades cut and the gripping members grip at the same time, i.e. when the tool is brought from the open position to the closed position in a single closing motion;

8. As claimed in any one of the preceding claims wherein the gripping surface on only one of the two component shear bodies involves, at least in part, the upper border (cutting-edge border) of the shear body shank, adjacent to the cutting edge;

9. As claimed in any preceding claim wherein the co-acting gripping surfaces and gripping members match one another closely,

10. As claimed in any preceding claim wherein the gripping members are not detachable or separate from the device;

11. As claimed in any preceding claim wherein the gripping members may be mounted along part or all of the shank of the tool;

12. As claimed in any preceding claim wherein the tool may be provided with more than one set of gripping elements, and where the two or more sets of co-acting gripping members may exist on the same planar side of the tool;

13. As claimed in 4 wherein two or more sets of co-acting gripping members are provided, but where they are mounted on opposite planar sides of the tool, thereby allowing both of the two cut halves of the cut object to be gripped by the tool at the same time;

14. As claimed in any preceding claim wherein the tool may be hand-operated or may be operated using a power source (e.g. a battery);

15. As claimed in any preceding claim wherein the pivot component is located at the handle's free end (as in a pair of tweezers);

16. As claimed in any preceding claim wherein the means of motion may be a pivot such as a hinge;

17. As claimed in any preceding claim wherein the pivot component is located at the blade-adjacent end of the handle (as in a pair of scissors);

18. As claimed in any preceding claim wherein the blade non-adj acent ends are joined on to one other (as found in the structure of a pair of tweezers);

19. As claimed in any preceding claim wherein a secondary means of motion may be provided in the tool (in the case of a second pivot, it may exist with its axis parallel to or collinear with the axis of the first pivot);

20. As claimed in any preceding claim wherein the lever arms (handles) may be located on the same side of the hub of the means of motion as the blade jaws and gripping members;

21. As claimed wherein the component blade edges, gripping members, shanks and lever arms may not exist in the same axial plane, and may also exist at an angle to one another, where this angle may be less than, greater than, or the same as 90 degrees;

22. As claimed in any preceding claim wherein the co-acting gripping members are similar in style;

23. As claimed in any preceding claim wherein the gripping surfaces contact and fit against one another when the device is in the closed position;

24. As claimed in any preceding claim wherein the lever arms may be located on the opposite side of the hub as the jaws and members;

25. As claimed in any preceding claim wherein a gripping member is provided on only one of the two component shear bodies, being designed to fit against and grip against a pre-existing structural feature on the second, opposing, shear body, e.g. a gripping surface intended to engage with and fit against the notch found on some styles of suture scissors (in effect the structural feature adopts the role of a second gripping member with a gripping surface);

26. As claimed in any preceding claim wherein the opposing active surfaces of the gripping members that come into contact with each other, and which perform the gripping function when the tool is in the closed position, are smooth, serrated or otherwise patterned/shaped to facilitate and optimise the gripping action;

27. As claimed in any preceding claim where the lever-arms (handles) may be rectilinear;

28. As claimed in any preceding claim wherein the lever-arms (handles) may be non-rectilinear, and may be provided with one or more bends between their free ends and their hub-adjacent hub-portions;

29. As claimed in any preceding claim wherein the said gripping members overlie one another;

30. As claimed in any preceding claim wherein the cutting blades generally overlie one another;

31. As claimed in any preceding claim wherein the level of the blade cutting edge on only one of the two co-acting shear bodies is the same as the level of the gripping surface on that same shear body;

32. As claimed in any preceding claim wherein the gripping members and/or cutting blades are disposed side by side;

33. As claimed in any preceding claim wherein the device incorporates a plurality of blade and gripping member arrangements;

34. A Grip-cut tool substantially as herein described, with reference to the embodiments, by way of example, as shown in the accompanying drawings.
